# EUROPEAN PATENT APPLICATION

(11) **EP 1 640 004 A1**
(43) Date of publication of application: **29.03.2006**
(21) Application number: 04090377.5
(22) Date of filing: 24.09.2004
(51) Int. Cl.: A61K 31/426, A61K 31/427, A61K 31/422, A61K 31/423, A61K 31/4709, A61K 31/44, A61P 35/04, A61P 35/00

(54) **Use of epothilones in the treatment of bone metastases and bone tumors or cancers**

(71) Applicant: Schering Aktiengesellschaft, 13353 Berlin (DE)
(72) Inventor: Klar, Ulrich, 13503 Berlin (DE); Hoffmann, Jens, 16567 Mühlenbeck (DE); Pietsch, Hubertus, 13127 Berlin (DE)

(57) **Abstract**

The present invention relates to the use of Epothilones in the treatment of bone metastases and bone tumors or cancers, more particularly in treating, preventing or alleviating bone metastasis in a cancer patient.

## Description

### Field of the Invention

The present invention relates to the use of Epothilones in the treatment of bone metastases and bone tumors or cancers, more particularly in treating, preventing or alleviating bone metastasis in a cancer patient.

### Background of the Invention

The epothilones represent a new class of microtubule stabilizing cytotoxic agents (see Gerth, K. et al., *J Antibiot.,* **1996,** 49, 560-3; or Hoefle et al., *Angew. Chem. [Applied Chem.],* 1996, 108, 1671-1673). These cytotoxic antimitotic agents, block the mitotic spindle of a proliferating cell by binding to the spindle-peptide tubulin, and thus cause apoptosis (K.-H. Altmann, *Curr. Opin. Chem. Biol.,* **2001,** 5, 424-431).

The natural products Epothilone A and B as well as some of their synthetic derivatives have recently found interest in connection with the treatment of cancer, and a lot of work has been done on their synthesis (K. Nicolaou et al., *Angew. Chem.,* **1998**, *110,* 2120-2153) and the synthesis of modified structures.

WO 99/07692, WO 99/02514, WO 99/67252, and WO 2004/014919 disclose Epothilone derivatives, their synthesis and pharmaceutical use.

WO 00/66589 deals with the synthesis and pharmaceutical use of Epothilone derivatives having an alkenyl-, alkynyl-, or a cyclic ether containing substituent at the 6(10)-position of the macrocyclic ring.

WO 00/49021 discloses Epothilone derivatives with a halogen substituent in the 16(3)-position and their synthesis.

WO 00/71521 discloses a method for the synthesis of olefinic Epothilones.

WO 98/25929 deals with the manufacture of libraries of Epothilone analogs.

WO 99/43320 mentions, in a very general manner, the use of Epothilones for the treatment of cancer.

WO 03/074053 describes the use of Epothilones and Epothilone analogs in the treatment of brain diseases associated with proliferative processes.

WO 04/050089 describes the use of conjugates of Epothilones and Epothilone derivatives (as effectors) with suitable saccharides as saccharide derivatives (as recognition units) in the treatment of proliferative or angiogenesis-associated processes.

WO 2004/012735 describes the use of conjugates of Epothilones and Epothilone derivatives (as effectors) with suitable biomolecules (as recognition units) in the treatment of proliferative or angiogenesis-associated processes.

WO 03/007924 describes the combination of Epothilones with a bisphosphonate, a platinum compound or a vasculostatic compound as use as combination therapy in the treatment of, *inter alia,* bone metastasis.

None of these publications, however, describe the use of Epothilones as monotherapy for the treatment of bone metastasis or bone tumors. In fact, there is no general disclosure or suggestion in the prior art that pharmaceutically effective amounts of an Epothilone directly decrease benign or malignant proliferative processes in bones. Thus the technical problem underlying the present invention is to provide compounds for the manufacture of medicaments for use in the treatment of bone metastases and bone tumors or cancers. The solution to this technical problem is achieved by using Epothilones as described below for the manufacture of said medicaments.

### Summary of the Invention

It has now unexpectedly been found that certain Epothilones can inhibit bone metastasis and growth of bone tumors or cancers, and thus show a beneficial effect in the treatment of malignant diseases metastasizing to the bones. Accordingly, the present invention provides the use of an Epothilone in the manufacture of medicaments for use as an inhibitor of bone metastasis and bone tumor growth and is thus useful for the treatment of malignant diseases metastasizing to the bones. The invention also provides a method of inhibiting bone metastasis and bone tumor growth in a patient in need of such treatment, which method comprises the administration to the said patient of an effective amount of an Epothilone.

The invention also relates to methods of treating bone metastasis by oral, parental, rectal, or local, preferably inhalational, intravenous, or intraperitoneal, most preferably intravenous administration of an Epothilone.

In a particular embodiment of the present invention, the medicament containing the Epothilone is used to treat, prevent, or alleviate bone metastasis. The bone metastasis results from cancer elsewhere in the body, for example, prostate cancer, breast cancer, lung cancer, melanoma, pancreatic cancer, colorectal cancer, ovarian cancer and brain cancer. In particular, the medicament is for treating, preventing or alleviating the symptoms of bone metastasis, more particularly in the treatment of bone metastasis in patients with cancer, even more particularly in the treatment of patients with breast and prostate cancer. The medicament may also be used to prevent or alleviate symptoms of pain associated with bone metastases and risk of pathological fractures and hypercalcemia.

In another particular embodiement of the present invention, the medicament containing the Epothilone is used to treat, prevent, or alleviate primary bone tumors or cancers, including benign tumors and malignant tumors such as osteosarcoma, chondrosarcoma, Ewing's tumor, Giant cell tumor of bone, and Chordoma.

For the purposes of the present invention, an Epothilone is defined as a cyclic molecule with a 16-membered ring and variable substituents and pharmaceutical activity as a cytostatic agent that binds to tubulin (Asnes et al., *Anal. Biochem.* **1979,** *98,* 64-73; Job et al., *Cellular Pharmacol.* **1993,** I (Suppl. I), S7-S10; Lichtner et al., *PNAS* **2001,** *98,* 11743-11748). An Epothilone also includes Epothilone conjugates such as those described in WO 04/050089 and WO 2004/012735 which are herein incorporated by reference.

The preferred group of Epothilones is that wherein the Epothilone molecule contains a lactone or a lactame moiety.

Preferred Epothilones for use in the present invention are compounds of the general formula: 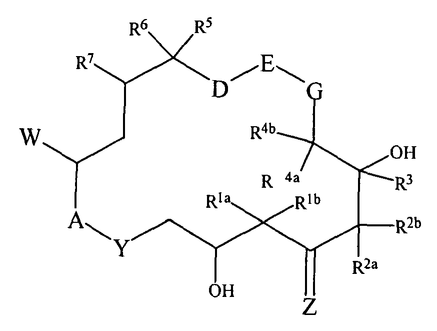 wherein:
- R^{1a} , R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
- R³: is hydrogen, C₁-C₁₀ alkyl, aryl, aralkyl;
- R^{4a}, R^{4b}: are each independently hydrogen, C₁-C₁₀ alkyl, aryl, aralkyl, or together form a -(CH₂)p- group where p is 2 to 5;
- R⁵: is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal;
- R⁶, R⁷: are each hydrogen, or together form an additional bond, or together form an epoxy function;
- G: is O or CH₂;
- D-E: together is a group -H₂C-CH₂-, -HC=CH-, -C≡C-, -CH(OH)- CH(OH)-, -CH(OH)-CH₂-, -CH₂-CH(OH)-, -CH₂-O-, -O-CH₂- or 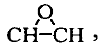 whereby if G is O then D-E cannot be CH₂-O; or
- D-E-G: together is a group H₂C-CH=CH
- W: is a group C(=X)R⁸, or is a bi- or tricyclic aromatic or heteroaromatic radical;
- X: is O, or two groups OR²⁰, or a C₂-C₁₀ alkylenedioxy group (which may be straight or branched), or H and the group OR⁹, or a group CR¹⁰R¹¹;
- R⁸: is hydrogen, C₁-C₁₀ alkyl, aryl, aralkyl, halogen, CN;
- R⁹: is hydrogen or a protecting group PG^{x};
- R¹⁰, R¹¹: are each independently hydrogen, C₁-C₂₀ alkyl, aryl, aralkyl, or together with the methylene carbon form a 5- to 7-membered carbocyclic ring;
- Z: is O or H and the group OR¹²;
- R¹²: is hydrogen or a protecting group PG^{z};
- A-Y: is a group O-C(=O), O-CH₂, CH₂-C(=O), NR²¹_C(=O), NR²¹-SO₂;
- R²⁰: is a C₁-C₂₀ alkyl group;
- R²¹: is hydrogen, or C₁-C₁₀ alkyl;
- PG^{x}, PG^{z}: is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers,
and / or as a pharmaceutically acceptable salt thereof.

### Preferred Embodiments

The term "therapeutically effective amount" as used herein refers to that amount of a compound of the invention which, when administered to an individual in need thereof, is sufficient to effect treatment, as defined below, for bone metastasis. The amount which constitutes a "therapeutically effective amount" will vary depending on the compound, the disease and its severity, and the age of the human to be treated, but can be determined routinely by one of ordinary skill in the art having regard to his own knowledge and to this disclosure.

"Treating" or "treatment" as used herein refers to the treatment of bone metastasis and/or bone tumors or cancers in an individual; and include:
(i) preventing the disease from recurring in an individual, in particular, when such individual is in need of further medicamentous treatment after a previous surgical or medicamentous therapy;
(ii) inhibiting the disease, i.e., arresting its development; or
(iii) relieving the disease, i.e., causing regression of the disease.

The term "alkyl" as used herein refers to straight or branched alkyl groups, e. g., methyl, ethyl, propyl, isopropyl, *n*-butyl, t-butyl, n-pentyl, neopentyl, heptyl, or decyl. Alkyl groups can be perfluorated or substituted by one to five substituents selected from the group consisting of halogen, hydroxy, C₁-C₄ alkoxy, or C₆-C₁₂ aryl (which can be substituted by one to three halogen atoms).

The term "alkenyl" as used herein refers to a straight or branched chain monovalent or divalent radical, containing at least one double bond and having from two to ten carbon atoms, e.g., ethenyl, prop-2-en-1-yl, but-1-enyl, pent-1-enyl, penta-1,4-dienyl, and the like.

The term "alkynyl" as used herein refers to a substituted or unsubstituted straight or branched chain monovalent or divalent radical, containing at least one triple bond and having from two to ten carbon atoms, e.g., ethynyl, prop-1-ynyl, but-1-ynyl, pent-1-ynyl, pent-3-ynyl, and the like.

Alkenyl and alkenyl groups can be substituted by one or more substituents selected from the group consisting of halogen, hydroxy, alkoxy, -CO₂H, -CO₂Alkyl, -NH₂, -NO₂, -N₃, - CN, C₁-C₂₀ acyl, or C₁-C₂₀ acyloxy.

The term "aryl" as used herein refers to an aromatic carbocyclic or heterocyclic moiety containing five to 14 ring atoms, e.g., phenyl, naphthyl, furyl, thienyl, pyridyl, pyrazolyl, pyrimidinyl, oxazolyl, pyridazinyl, pyrazinyl, chinolyl, or thiazolyl. Aryl groups can be substituted by one or more substituents selected from the group consisting of halogen, hydroxy, alkoxy, -CO₂H, -CO₂Alkyl, -NH₂, Alkyl-NH₂, C₁-C₂₀ alkyl-thiolanyl, -NO₂, - N₃, -CN, C₁-C₂₀ alkyl, C₁-C₂₀ acyl, or C₁-C₂₀ acyloxy. The heteroatoms can be oxidized, if this does not cause a loss of aromatic character, e. g., a pyridine moiety can be oxidized to give a pyridine N-oxide.

The term "aralkyl" as used herein refers to a group which can contain up to 14 atoms in the aryl ring (preferred five to ten) and one to eight carbon atoms in the alkyl chain (preferred one to four), e.g., benzyl, phenylethyl, naphthylmethyl, naphthylethyl, furylmethyl, thienylethyl, or pyridylpropyl. The rings can be substituted by one or more substituents selected from the group consisting of halogen, hydroxy, alkoxy, -CO₂H, - CO₂Alkyl, -NH₂, -NO₂, -N₃, -CN, C₁-C₂₀ alkyl, C₁-C₂₀ acyl, or C₁-C₂₀ acyloxy.

The protecting groups PG can be alkyl- and/or aryl-substituted silyl moieties, C₁-C₂₀ alkyl, C₄-C₇ cycloalkyl, which may contain an oxygen atom in the ring, aryl, aralkyl, C₁C₂₀ acyl, aroyl, alkyl- or arylsulfonyl. Groups which can be easily be removed from the molecule are preferred, e.g., methoxymethyl, methoxyethyl, polyethylene glycol, ethoxyethyl, tetrahydropyranyl, tetrahydrofuranyl, trimethylsilyl, triethylsilyl, t-butyldimethylsilyl, tribenzylsilyl, triisopropylsilyl, benzyl, *p*-nitrobenzyl, *p-*methoxybenzyl, as well as alkylsulfonyl or arylsulfonyl. Preferred acyl groups are formyl, acetyl, propionyl, pivaloyl, butyryl, or benzoyl, which all can be substituted by one or more amino and/or hydroxy moieties.

A preferred group is compounds of the general formula as given above, wherein A-Y is O-C(=O); D-E is H₂C-CH₂; G is CH₂; Z is O; R^{1a}, R^{1b} are both C₁-C₁₀ alkyl or form together a -(CH₂)ₚ- group where p is 2 to 3; R^{2a}, R^{2b} are each independently hydrogen, C₁-C₁₀ alkyl, C₂-C₁₀ alkenyl, or C₂-C₁₀ alkynyl; R³ is hydrogen; R^{4a}, R^{4b} are each independently hydrogen or C₁-C₁₀ alkyl; R⁵ is C₁-C₁₀ alkyl.

Another preferred group is compounds of the general formula as given above, wherein R^{2a}, R^{2b} are each independently hydrogen, C₂-C₁₀ alkenyl or C₂-C₁₀ alkynyl; R⁶, R⁷ form an epoxy function or together form an additional bond; W is a 2-Methyl-benzothiazol-5-yl radical or a 2-Methyl-benzoxazol-5-yl radical or a Quinoline-7-yl radical.

Of this group, a preferred subgroup is compounds selected from the following: (4S,7R,8S,9S,13E/Z,-16S(E))-4,8-dihydroxy-16-(2-methyl-benzoxazol-5-yl)-1-oxa 5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-methyl-benzoxazol-5-yl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(2-methyl-benzothiazol-5-yl)-5-oxa-5,5,9,13-tetramethyl-7-(prop-2-en-1-yl)-cyclohexadec-13-ene-2,6-dione;
(1S/R,3S(E),7S,10R,10R,11R,12S,16R/S)-7,11-dihydroxy-10-(prop-2-en-1-yl)-3-(1-methyl-2-(2-methyl-benzothiazol-5-yl)-8,8,12,16-tetramethyl-4,17-dioxabicyclo[14.1.0] heptadecane-5,9-dione;
(4S,7R,8S,9S, 13E/Z, 16S(E))-4,8-dihydroxy-16-(2-methyl-benzothiazol-5-yl). 1 -oxa-9,13-dimethyl-5,5-(1,3-trimethylen)-7-(prop-2-en-1-yl)-cyclohexadec-13-ene-2,6-dione;
(1S/R,3S(E),7S, 10R,11R,12S,16R/S)-7,11-dihydroxy-10-(prop-2-en-1-yl)-3-(1-methyl-2-(2-methyl-benzothiazol-5-yl)-12,16-dimethyl-8,8-(1,3-trimethylen)-4,17-dioxabicyclo [14.1.0]heptadecane-5,9-dione;
(4S,7R,8S,9S, 13E/Z, 16S(E))-4,8-dihydroxy-16-(2-methyl-benzothiazol-5-yl)-1 -oxa-5,5,9,13-tetramethyl-7-(prop-2-in-1-yl)-cyclohexadec-13-ene-2,6-dione;
(1S/R,3S(E),7S,11R,12R,12S,16R/S)-7,11-dihydroxy-10-(prop-2-in-1-yl)-3-(1-methyl-2-(2-methyl-benzothiazol-5-yl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(chinolin-2-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(chinolin-2-yl)-8,8,12,16-tetramethyl-4,17-dioxabicyclo[14.1.0]heptadecane-5,9-dione;
(1S,3S,7S,10R, 11S, 12S, 16R)-7,11-dihydroxy-3-(2-methyl-benzothiazol-5-yl)- 10-(prop-2-en-1-yl)-8,8,12,16-tetramethyl-4,17-dioxabicyclo[14.1.0]heptadecane-5,9-dione;
(4S,7R,8S,9S, 13E/Z, -16S(E))-4,8-dihydroxy-16-(2-methyl-benzothiazol-5-yl)-1-aza 5,5,9,13-tetramethyl-7-(prop-2-en-1-yl)-cyclohexadec-13-ene-2,6-dione; and
(1S/R,3S(E),7S,10R,11R,12S,16R/S)-7,11-dihydroxy-10-(prop-2-en-1-yl)-3-(1-methyl-2-(2-methyl-benzothiazol-5-yl)-8,8,12,16-tetramethyl-4-aza-17-oxabicyclo[14.1.0] heptadecane-5,9-dione.

Another preferred group of compounds has the general formula as given above, wherein R^{2a}, R^{2b} are each independently hydrogen, or C₁-C₁₀ alkyl; R⁶, R⁷ form an epoxy function, or form an additional bond; W is a group C(=X)R⁸; X is a group CR¹⁰R¹¹; R⁸ is hydrogen, halogen, C₁-C₁₀ alkyl; R¹⁰, R¹¹ are hydrogen/2-methyl-thiazol-4-yl, hydrogen/2-pyridyl, hydrogen/2-methylamine-thiazol-4-yl, or hydrogen/2-methylsulfanyl-thiazol-4-yl .

Of this group, a preferred subgroup is compounds selected from the following: (4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8-(1,3-trimethylen)-12,16-dimethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-methyl-4-thiazolyl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8,10,12,16-pentamethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S, 10R,11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S, 10R, 11S,12S,16R/S)-7,11-dihydroxy-3-(1-fluor-2-(2-methyl-4-thiazolyl)ethenyl)-8,8-(1,3-trimethylen)-10,12,16-trimethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(Z),7S, 10R, 11S,12S,16R/S)-7,11-dihydroxy-3-(1-fluor-2-(2-methyl-4-thiazolyl)ethenyl)-8,8-( 1,3-trimethylen)-12,16-dimethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,7,9,13-pentamethyl-cyclohexadec-13-ene-2,6-dione;
(1S/R,3S(Z),7S, 10R, 11S,12S,16R/S)-7,11-dihydroxy-3-(1-chlor-2-(2-methyl-4-thiazolyl)ethenyl)-8,8,10,12,16-pentamethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R, 3S(Z), 7S, 10R, 11S,12S,16R/S)-7,11-dihydroxy-3-(1-chlor-2-(2-methyl-4-thiazolyl)ethenyl)-8,8, 12, 16-tetramethyl-10-ethyl-4,17-dioxabicyclo[14.1.0] heptadecane-5,9-dione;
(4S,7R, 8S, 9S,13E/Z,16S(Z))-4,8-dihydroxy-16-(1-chlor-2-(2-methyl-4-thiazolyl)ethenyl)-1-oxa-5,5,9,13-tetramethyl-7-propyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-propyl-4,17-dioxabicyclo[14.1.0] heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S, 7R, 8S,9S,13E/Z,16S(E))-4,8-dihydroxy-16-(1-methyl-2-(2-pyridyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-pyridyl)ethenyl)-8,8-(1,3-trimethylen)-12,16-dimethyl-4,17-dioxabicyclo[14.1.0] heptadecane-5,9-dione;
(4S,7R,8S,9S, 13E/Z, 16S(E))-4,8-dihydroxy-16-(1-methyl-2-(2-pyridyl)ethenyl)-1-oxa-5,5-(1,3-trimethylen)-7,9,13-trimethyl-cyclohexadec-13-ene-2,6-dione;
(1S/R,3S(E),7S,10R,11R,12S,16R/S)-7,11-dihydroxy-3-(1-methyl-2-(2-pyridyl)ethenyl)-8,8-(1,3-trimethylen)-10,12,16-trimethyl-4,17-dioxabicyclo[14.1.0]heptadecane-5,9-dione;
(4S,7R,8S,9S,13E/Z, 16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,1 1-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S, 10R, 11S,12S,16R/S)-7,11-dihydroxy-3-(1-fluor-2-(2-methyl-4-thiazolyl)ethenyl)-8,8-(1,3-trimethylen)-10,12,16-dimethyl-4,17-dioxabicyclo[14.1.0] heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl) ethenyl)-1-oxa-5,5,9,13-tetramethyl-7-ethyl-cyclohexadec-13-ene-2,6-dione; and
(1S/R,3S(Z),7S, 10R, 11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0] heptadecane-5,9-dione.

Another preferred group is compounds of the general formula as given above, wherein R^{2a}, R^{2b} are each independently hydrogen, C₂-C₁₀ alkenyl or C₂-C₁₀ alkynyl; R⁶, R⁷ form an epoxy function or together form an additional bond; W is a group C(=X)R⁸; X is a group CR¹⁰R¹¹; R⁸ is hydrogen, halogen, C₁-C₁₀ alkyl; R¹⁰, R¹¹ are hydrogen/2-methylthiazol-4-yl or hydrogen/2-pyridyl.

Of this group, a preferred subgroup is compounds selected from the following:
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(but-3-in-1-yl)-cyclohexadec-13-ene-2,6-dione;
(1S/R,3S(E),7S,10R,11R,12S,16R/S)-7,11-dihydroxy-10-(but-3-in-1-yl)-3-(1-methyl-2-(2-pyridyl)ethenyl)-8,8,12,16-tetramethyl-4,17-dioxabicyclo[14.1.0]heptadecane-5,9-dione;
(1S/R,3S(E),7S,10R,11R,12S,16R/S)-7,11-dihydroxy-10-(but-3-en-1-yl)-3-(1-methyl-2-(2-pyridyl)ethenyl)-8,8, 12, 16-tetramethyl-4, 17-dioxabicyclo[14.1.0]heptadecane-5,9-dione;
(1S/R,3S(E),7S,10R,11R,12S,16R/S)-7,11-dihydroxy-10-(but-3-en-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13 E/Z,16S(Z))-4,8-dihydroxy-16-(1-fluor-2-(2-methylthiazol-4-yl)ethenyl)-1-oxa-5,5,9,13-tetramethyl-7-(prop-2-in-1-yl)-cyclohexadec-13-ene-2,6-dione;
(1S/R,3S(Z),7S,10R,11R,12S,16R/S)-7,11-dihydroxy-10-(prop-2-in-1-yl)-3-(1-fluor-2-(2-methylthiazol-4-yl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-en-1-yl)-cyclohexadec-13-ene-2,6-dione; and
(1S/R,3S(Z),7S,10R,11R,12S,16R/S)-7,11-dihydroxy-10-(prop-2-en-1-yl)-3-(1-fluor-2-(2-methylthiazol-4-yl)ethenyl)-8,8,12,16-tetramethyl-4,17-dioxabicyclo[14.1.0] heptadecane-5,9-dione.

Also preferred are the natural compound Epothilone B and compounds of the general formula as given above wherein A-Y is NR²¹-C=O.

The synthesis of the compounds listed above is described in the international patent applications WO 99/07692, WO 00/49021, WO 03/041068, WO 03/041063, WO 00/66589, WO 04/050089 and WO 2004/012735 which are incorporated herein by reference.

For the use according to the invention, the compounds can be formulated by methods known in the art. Compositions for the oral, rectal, parenteral or local application can be prepared in the form of tablets, capsules, granulates, suppositories, implantates, sterile injectable aqueous or oily solutions, suspensions or emulsions, aerosols, salves, creams, or gels, retard preparations or retard implantates. The compounds may also be administered by implantable dosing systems.

The pharmaceutical active compound(s) can thus be mixed with adjuvants known in the art, such as gum arabic, talcum, starch, mannitol, methyl cellulose, lactose, surfactants such as tweens® or myrj®, magnesium stearate, aqueous or non-aqueous carriers, paraffin derivatives, wetting agents, dispersing agents, emulsifiers, preservatives, and flavors.

The compounds can be used in the form of their clathrates of α-, β-, or γ-cyclodextrin or of substituted α-, β-, or γ-cyclodextrines, or in the form of a liposomal composition, in particular a liposomal composition comprising a polyethyleneglycol(PEG)-derivatized lipid.

The invention also relates to pharmaceutical compositions containing one or more of the pharmaceutically active compounds listed above, and their use for the treatment and in the methods in accordance with the present invention. Preferably, one dose unit of these compositions contains about 0.01-100 mg of the pharmaceutically active compound(s). The dosage for the use according to the invention for a human is about 0.01-100 mg per day; a preferred dosage is about 0.02-70 mg per day; a more preferred dosage is about 0.04-40 mg per day.

Compounds of the present invention have demonstrated positive results in bone metastasis animal models. The compounds of the present invention can be tested for utility through clinical trials, wherein the compounds are administered to human bone metastasis patients and the endpoint determines whether the compounds have any effect on bone metastasis. The beneficial effects of Epothilones to reduce skeletal-related events (SREs) in cancer patients with a history of metastatic bone disease, can also be determined directly through the results of these studies or by changes in the study design known to a person skilled in the art. SREs are defined as pathological bone fracture events, spinal cord compression events, surgery to bone, radiation therapy to bone and a change of antineoplastic therapy to treat bone pain.

The present invention will be further illustrated in the following Examples.

### Example 1 - Inhibition of Bone Metastasis Using an Epothilone (Bone Metastasis Model)

### Model:

Human breast cancer MDA MB 231, injected to NMRI nude mice intracardially

### Test Compound:

7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione

### Experimental Design:

Four days before tumor cells implantation, the baseline bone structure of the mice was determined by X-ray. 30 NMRI nude mice received 500,000 of MDA-MB 231 breast cancer tumor cells intracardially (Day 0). The tumor cells metastasized to the bones and the growth of the tumor in the bones lead to bone destruction. After the period of 10 days (Day 10), 10 of the 29 animals were treated with the Test Compound (10 mg/kg, i.v.). The size and number of bone metastases were monitored 4 days before tumor cell implantation (baseline) and on Day 22 in each animal using the X-ray technology described below.

### Investigation of Bone Metastasis on Mice with Mammomat 3000 (Siemens) (X-Ray Technology):

### Preparation of the Animals (anaesthesia, fixation)

The mice underwent an anaesthesia with a mixture of Rompun ( Rompun 2% Bayer) diluted with sodium chloride solution( Braun) by 1:10 and Ketavet ( Pharmacia GmbH 100mg/ ml), diluted by 1:5 with isotonic sodium chloride solution. For application of the narcotics, 1ml sterile syringes (Codan Medical) and cannula (Braun Sterican, size 18) were used.

Once the tolerance stage was reached, the mice were attached to a 1 mm strong acrylic glass and additionally taped with a self-adhesive tape (sticky side facing the animal). The acrylic glass served as surface on which the animals underwent the X-ray.

### Instrumental Equipment/ Mammomat for Monochromatic X-Ray

At the console of the Mammomat (run by the software "Detector" (lfg) on Windows NT platform), the standard setting of 35kV and 400mAs was adjusted. The setting was chosen in such a way to allow a good contrasting of the entire skeletal system and visibility of finest changes/aberrations of the bone structure and morphology. The resolution of the systems lies at 0,27 µm.

After initializing the Software the module "Zoomed Modus" was chosen for the detector and the ROIs 2-3 were selected in accordance with the previously selected positioning of the mouse. The regulator Image High was adjusted to 2000.

The actual measurements were started as soon as the Offset measurings and the reference measurings had been performed. The flexible table was brought into position (80 mm) and was advanced at a speed of 25,9 mm/sec. The final position of the table was chosen in such a way that the entire body of the mouse could be X-rayed all at once during the examination. The reference measuring was subtracted from each gained Image and was stored as a DICOM-file or as a BMP-file.

### Results

Analysis of the treatment was performed on Day 22 by evaluating twice the constructed exposures of the mice and searching for destructive processes of the skeletal system (focussing mainly on the long bones, spine and pelvis). The extend/area of the destructive processes/bone metastasis were determined with a special DICOM-Software (based on Microsoft Excel).

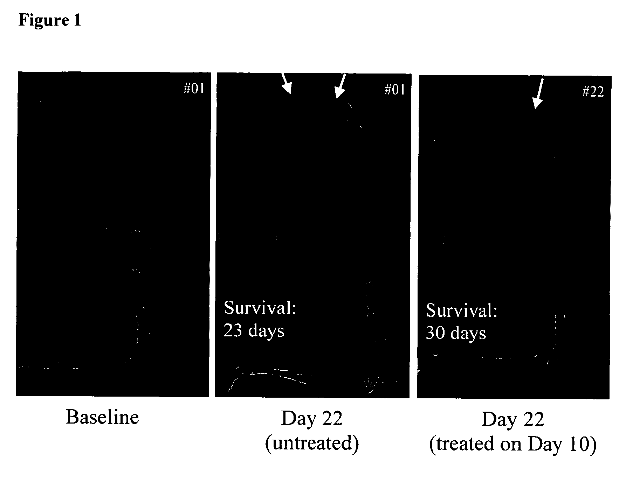

Results shown in **Figure 1** show that the Test Compound inhibited bone metastasis in the *in vivo* model and that a prolongation of life time was observed. In fact, the size of the bone metastases were reduced after just 12 days following a single treatment and Mean Survival Time was improved. As can be seen in **Table 1**, treatment with the Test Compound resulted in a 5.9 mm² Mean Tumor Area compared to 14.0 mm² without treatment. The overall Mean Survival Time rose from 23.4 days without treatment to 31.6 days following a single application of the Test Compound in the treatment group.

**Table 1**

| Treatment | Mean Bone Tumor Area | Mean Survival Time |
|---|---|---|
| Control: none | 14 mm² | 23.4 days |
| Test compound: 10mg/kg on Day 10 | 5.9 mm² | 31.6 days |

### Description of the Figures

**Figure 1** visualises the efficacy in the bone metastasis model. The baseline bone structure of the mice was determined by X-ray (Animal #01, left). MDA-MB 231 breast cancer tumor cells were implanted intracardially into nude mice on Day 0 (i.e. four days after baseline). Treatment was started on Day 10 when first signs of bone metastasis were observed. The same untreated animal, whose survival time of 23 days is close to the mean survival time (23.4 days) of the untreated control group, has developed severe bone metastasis on Day 22 (see white arrows, middle). Animal #22 represents a typical member of the treatment group whose survival time of 30 days is close to the mean survival time (31.6 days) in the treatment group. Size and amount of bone metastasis are reduced. The Test Compound was given in the indicated dose. The size of the bone metastases after Day 22 with and without treatment is shown.

## Claims

1. Use of an Epothilone for the preparation of a medicament for use as an inhibitor of bone metastasis or tumor growth.

2. Use according to claim 1 wherein the medicament is for treating, preventing or alleviating bone metastasis.

3. Use according to claim 1 wherein the medicament is for treating, preventing or alleviating bone tumor growth.

4. Use according to claim 3 wherein the medicament is used to treat, prevent, or alleviate primary bone tumors or cancers including benign tumors and malignant tumors such as osteosarcoma, chondrosarcoma, Ewing's tumor, Giant cell tumor of bone, and Chordoma.

5. Use according to claim 2 wherein the medicament is for treating, preventing or alleviating bone metastasis in a cancer patient.

6. Use of any one of claims 1-5, wherein the Epothilone contains a lactone or a lactame moiety.

7. Use of any one of claims 1-5 wherein the Epothilone is a compound of the general formula: 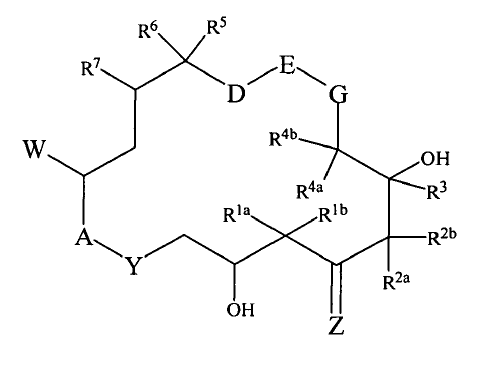 wherein
R^{1a}, R^{1b} are each independently hydrogen, C₁-C₁₀ alkyl, aryl, aralkyl, or together form a -(CH₂)ₘ-group where m is 2 to 5;
R^{2a}, R^{2b} are each independently hydrogen, C₁-C₁₀ alkyl, aryl, aralkyl, or together form a -(CH₂)ₙ-group where n is 2 to 5, or are C₂-C₁₀ alkenyl or C₂-C₁₀alkynyl;
R³ is hydrogen, C₁-C₁₀ alkyl, aryl, aralkyl;
R^{4a}, R^{4b} are each independently hydrogen, C₁-C₁₀ alkyl, aryl, aralkyl, or together form a-(CH₂)ₚ- group where p is 2 to 5;
R⁵ is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal;
R⁶ R⁷ are each hydrogen, or together form an additional bond, or together form an epoxy function;
G is O or CH₂;
D-E together is a group -H₂C-CH₂-, -HC=CH-, -C≡C-, -CH(OH)- CH(OH)-, -CH(OH)-CH₂-, -CH₂-CH(OH)-, -CH₂-O-, -O-CH₂- or 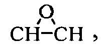 whereby if G is O then D-E cannot be CH₂-O; or
D-E-G together is a group H₂C-CH=CH;
W is a group C(=X)R⁸, or is a bi- or tricyclic aromatic or heteroaromatic radical;
X is O, or two groups OR²⁰, or a C₂-C₁₀ alkylenedioxy group (which may be straight or branched), or H and the group OR⁹, or a group CR¹⁰R¹¹;
R⁸ is hydrogen, C₁-C₁₀ alkyl, aryl, aralkyl, halogen, CN;
R⁹ is hydrogen or a protecting group PG^{x};
R¹⁰, R¹¹ are each independently hydrogen, C₁-C₂₀ alkyl, aryl, aralkyl, or together with the methylene carbon form a 5- to 7-membered carbocyclic ring;
Z is O or H and the group OR¹²;
R¹² is hydrogen or a protecting group PG^{Z};
A-Y is a group O-C(=O), O-CH₂, CH₂-C(=O), NR²¹-C(=O), or NR²¹- SO₂;
R²⁰ is a C₁-C₂₀ alkyl group;
R²¹ is hydrogen, or C₁-C₁₀ alkyl;
PG^{x}, PG^{z} is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl) diarylsilyl, or tri(aralkyl)silyl;
as a single stereoisomer or a mixture of different stereoisomers, and/or as a pharmaceutically acceptable salt thereof.

8. The use of claim 7, wherein
A-Y is O-C(=O);
D-E is H₂C-CH₂;
G is CH₂;
Z is O;
R^{1a}, R^{1b} are both C₁-C₁₀ alkyl or together form a-(CH₂)ₘ- group where m is 2 or 3;
R^{2a}, R^{2b} are each independently hydrogen, C₁-C₁₀ alkyl, C₂-C₁₀ alkenyl, or C₂-C₁₀ alkynyl;
R³ is hydrogen;
R^{4a}, R^{4b} are each independently hydrogen or C₁-C₁₀ alkyl;
R⁵ is C₁-C₁₀ alkyl.

9. The use of any one of claims 7 or 8, wherein
R^{2a}, R^{2b} are each independently hydrogen, C₂-C ₁₀ alkenyl or C₂-C₁₀ alkynyl;
R⁶, R⁷ together form an epoxy function or an additional bond; and
W is a 2-Methyl-benzothiazol-5-yl radical, a 2-Methyl-benzoxazol-5-yl radical, or a Quinoline-7-yl radical.

10. The use of claim 7, wherein the compound is selected from the group consisting of:
(4S,7R,8S,9S, 13E/Z, 16S(E))-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-1 0-(prop-2-en-1-yl)-3-(1-methyl-2-(2-methyl-benzoxazol-5-yl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-oxa-9,13-dimethyl-5, 5-(1,3-trimethylen)-7-(prop-2-en-1-yl)-cyelohexadec-13-ene-2,6-dione;
(1S/R,3S(E),7S,10R,11R,12S,16R/S)-7,11-dihydroxy-10-(prop-2-en-1-yl)-3-(1-methyl-2-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S(E),7S, 10R, 11R, 12S, 16R/S)-7,11-dihydroxy-10-(prop-2-in-1-yl)-3-(1-methyl-2-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(chinolin-2-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-l-yl)-3-(1-methyl-2-(chinolin-2-yl)-8,8,12,16-tetramethyl-4,17-dioxabicyclo[14.1.0] heptadecane-5,9-dione;
(1S,3S,7S,10R, 11S,125,16R)-7,11-dihydroxy-3-(2-methyl-benzothiazol-5-yl)-10-(prop-2-en-1-yl)-8,8,12,16-tetramethyl-4,17-dioxabicyclo[14.1.0] heptadecane-5,9-dione;
(4S,7R,8S,9S, 13E/Z, 16S(E))-4,8-dihydroxy-16-(2-methyl-benzothiazol-5-yl)-1-aza-5,5,9,13-tetramethyl-7-(prop-2-en-1-yl)-cyclohexadec-13-ene-2,6-dione; and
(1S/R,3S(E),7S,10R,11R,12S,16R/S)-7,11-dihydroxy-10-(prop-2-en-1-yl)-3-(1-methyl-2-(2-methyl-benzothiazol-5-yl)-8,8,12,16-tetramethyl-4-aza-17-oxabicyclo[14.1.0]heptadecane-5,9-dione.

11. The use of any one of claims 7 or 8, wherein
R^{2a}, R^{2b} are each independently hydrogen, or C₁-C₁₀ alkyl;
R⁶ R⁷ together form an epoxy function or an additional bond;
W is a group C(=X)R⁸;
X is a group CR¹⁰R¹¹;
R⁸ is hydrogen, halogen, or C₁-C₁₀ alkyl; and
R¹⁰, R¹¹ are hydrogen/2-methyl-thiazol-4-yl, hydrogen/2-pyridyl, hydrogen/2-methylamine-thiazol-4-yl, or hydrogen/2-methylsulfanyl-thiazol-4-yl .

12. The use of claim 7, wherein the compound is selected from the group consisting of:
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8-(1,3-trimethylen)-12,16-dimethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-propyl-cyclohexadec-13-ene-2,6-dione;
(1S/R,3S(E),10R,11R,12S,16R/S)-7,11-dihydroxy-10-propyl-3-(1-methyl-2-(2-methyl-4-thiazolyl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,7,9,13-pentamethyl-cyclohexadec-13-ene-2,6-dione;
(1S/R,3S(Z),7S, 10R,11S,12S,16R/S)-7,11-dihydroxy-3-(1-fluor-2-(2-methyl-4-thiazolyl)ethenyl)-8,8,10,12,16-pentamethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S, 10R, 11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S, 10R, 11S,12S,16R/S)-7,11-dihydroxy-3-(1-fluor-2-(2-methyl-4-thiazolyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(Z),7S,10R, 11S,12S,16R/S)-7,11-dihydroxy-3-(1-fluor-2-(2-methyl-4-thiazolyl)ethenyl)-8,8-(1,3-trimethylen)-12,16-dimethyl-10-ethyl-4,17-dioxabicyclo[14.1.0]heptadecane-5,9-dione;
(4S, 7R, 8S,9S,13E/Z,16S (Z))-4,8-dihydroxy-16-(1-chlor-2-(2-methyl-4-thiazolyl)ethenyl)-1-oxa-5,5,7,9,13-pentamethyl-cyclohexadec-13-ene-2,6-dione;
(1S/R,3S(Z),7S,10R, 11S,12S,16R/S)-7,11-dihydroxy-3-(1-chlor-2-(2-methyl-4-thiazolyl)ethenyl)-8,8,10,12,16-pentamethyl-4,17-dioxabicyclo[14.1.0] heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R, 11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo [14.1.0] heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-propyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-propyl-4,17-dioxabicyclo[14.1.0] heptadecane-5,9-dione;
(4S,7R,8S,9S, 13E/Z, 16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R, 12S, 16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,-16S(E))-4,8-dihydroxy-16-(1-methyl-2-(2-pyridyl)ethenyl) 1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-pyridyl)ethenyl)-8,8-(1,3-trimethylen)-12,16-dimethyl-4,17-dioxabicyclo[14.1.0] heptadecane-5,9-dione;
(4S,7R,8 S,9S,13E/Z,16S(E))-4,8-dihydroxy-16-(1-methyl-2-(2-pyridyl)ethenyl)-1-oxa-5,5-(1,3-trimethylen)-7,9,13-trimethyl-cyclohexadec-13-ene-2,6-dione;
(1S/R,3S(E),7S,10R,11R,12S,16R/S)-7,11-dihydroxy-3-(1-methyl-2-(2-pyridyl)ethenyl)-8, 8-(1,3-trimethylen)-10,12,16-trimethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl-1-oxa-5,5,9,13-tetramethyl-7-propyl-cyclohexadec-13-ene-2,6-dione;
(1S/R,3S(E),7S, 10R, 11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-dimethyl-4,17-dioxabicyclo[14.1.0] heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1 S/R,3S(Z),7S, 10R, 11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione; and
(1S/R,3S(Z),7S, 10R, 11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0] heptadecane-5,9-dione.

13. The use of any one of claims 7 or 8, wherein
R^{2a}, R^{2b} are each independently hydrogen, C₂-C₁₀ alkenyl or C₂-C₁₀ alkynyl;
R⁶, R⁷ together form an epoxy function or an additional bond;
W is a group C(=X)R⁸;
X is a group CR¹⁰R¹¹;
R⁸ is hydrogen, halogen, or C₁-C₁₀ alkyl; and
R¹⁰, R¹¹ are hydrogen/2-methylthiazol-4-yl or hydrogen/2-pyridyl.

14. The use of claim 7, wherein the compound is selected from the group consisting of:
(4S,7R,8S,9S,13E/Z,16S(E))-4,8-dihydroxy-16-(1-methyl-2-(2-pyridyl)ethenyl) 1-oxa-5,5,9,13-tetramethyl-7-(prop-2-in-1-yl)-cyclohexadec-13-ene-2,6-dione;
(1S/R,3S(E),7S, 10R,11R,12S,16R/S)-7,11-dihydroxy-10-(prop-2-in-1-yl)-3-(2-(2-pyridyl)ethenyl)-8,8,12,16-tetramethyl-4,17-dioxabicyclo[14. 1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-pyridyl)ethenyl)-8,8,12,16-tetramethyl-4,17-dioxabicyclo[14.1.0] heptadecane-5,9-dione;
(4S,7R,8S,9S, 13E/Z, 16S(E))-4,8-dihydroxy-16-(1-methyl-2-(2-pyridyl)ethenyl)-1-oxa-5,5,9,13-tetramethyl-7-(but-3-in-1-yl)-cyclohexadec-13-ene-2,6-dione;
(1S/R,3S(E),7S,10R,11R,12S,16R/S)-7,11-dihydroxy-10-(but-3-in-1-yl)-3-(1-methyl-2-(2-pyridyl)ethenyl)-8,8,12,16-tetramethyl-4,17-dioxabicyclo[14.1.0] heptadecane-5,9-dione;
(1S/R,3S(E),7S,10R,11R,12S,16R/S)-7,11-dihydroxy-10-(but-3-en-1-yl)-3-(1-methyl-2-(2-pyridyl)ethenyl)-8,8,12,16-tetramethyl-4,17-dioxabicyclo[14.1.0] heptadecane-5,9-dione;
(1S/R,3S(E),7S,10R,11R,12S,16R/S)-7,11-dihydroxy-10-(but-3-en-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-in-1-yl)-cyclohexadec-13-ene-2,6-dione;
(1S/R,3S(Z),7S,10R, 11R,12S,16R/S)-7,11-dihydroxy-10-(prop-2-in-1-yl)-3-(1-fluor-2-(2-methylthiazol-4-yl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-en-1-yl)-cyclohexadec-13-ene-2,6-dione; and
(1S/R,3S(Z),7S,10R,11R,12S,16R/S)-7,11-dihydroxy-10-(prop-2-en-1-yl)-3-(1-fluor-2-(2-methylthiazol-4-yl)ethenyl)-8,8,12,16-tetramethyl-4,17-dioxabicyclo[14.1.0] heptadecane-5,9-dione.

15. The use of claim 7, wherein:
A-Y is NR²¹-C(=O)

16. The use of any one of claims 1 to 6 wherein the Epothilone is Epothilone B

17. A method of treating bone metastasis or tumors comprising administering to an individual in need thereof a therapeutically effective amount of an Epothilone as defined in any one of claims 6 to 16.

18. A method according to claim 17 of treating, preventing or alleviating bone metastasis.

19. A method according to claim 17 of treating, preventing or alleviating bone tumor growth.

20. A method according to claim 18 of treating, preventing or alleviating bone metastasis in a cancer patient.

21. The use or the method according to any one of claims 1 to 20, wherein the medicament or the Epothilone is to be administered orally, parenterally, rectally, or locally.

22. The use or method according to claim 21 wherein the medicament or the Epothilone is administered intravenously.
